(19) Europäisches Patentamt
European Patent Office
Office européen des brevets

(11) **EP 3 225 175 A1**

(12) **EUROPEAN PATENT APPLICATION**

(43) Date of publication:
**04.10.2017 Bulletin 2017/40**

(51) Int Cl.:
**A61B 17/06** (2006.01)     A61B 17/00 (2006.01)

(21) Application number: **16163498.5**

(22) Date of filing: **01.04.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Beauty-Com Biotechnology Co., Ltd
Taichung City 403 (TW)**

(72) Inventor: **LIN, Chun-Jung
407 Taichung City (TW)**

(74) Representative: **Viering, Jentschura & Partner
mbB
Patent- und Rechtsanwälte
Kennedydamm 55 / Roßstrasse
40476 Düsseldorf (DE)**

(54) **SURGICAL SUTURE**

(57)     A surgical suture (100) is provided, which includes a hollow cone (10, 40, 50) and a thread (20). The cone (10, 40, 50) has a front opening (12, 42, 52) and a rear opening (14, 44, 54) at two opposite ends thereof, wherein a diameter (D1) of the front opening (12, 42, 52) is smaller than a diameter (D2) of the rear opening (14, 44, 54). The thread (20) passes through the cone (10, 40, 50), and integrally includes at least two protrusions (22) thereon, which are separated from each other by a certain distance. Each of the protrusions (22) has a width (W) in a radial direction of the thread (20), wherein the width (W) is greater than a diameter (D) of the thread (20), and is smaller than the diameter (D2) of the rear opening (14, 44, 54). Whereby, with the integrally provided protrusions (22), the cones (10, 40, 50) can be located on the corresponding positions of the thread (20).

FIG. 4

## Description

## BACKGROUND OF THE INVENTION

### 1. Technical Field

The present invention relates generally to a surgical thread, and more particularly to a surgical suture for surgery.

### 2. Description of Related Art

Aesthetic medicine has become a popular industry in recent years, wherein the face-lift surgery is especially the most performed. Thread lift surgery is developed to satisfy such huge demand, which reduces severe traumas and scars caused by the traditional lift surgery, and effectively retards the skin sagging and aging. In light of this, manufacturers in the related industry have invested much effort in researching and developing the medical materials for thread lift surgery.

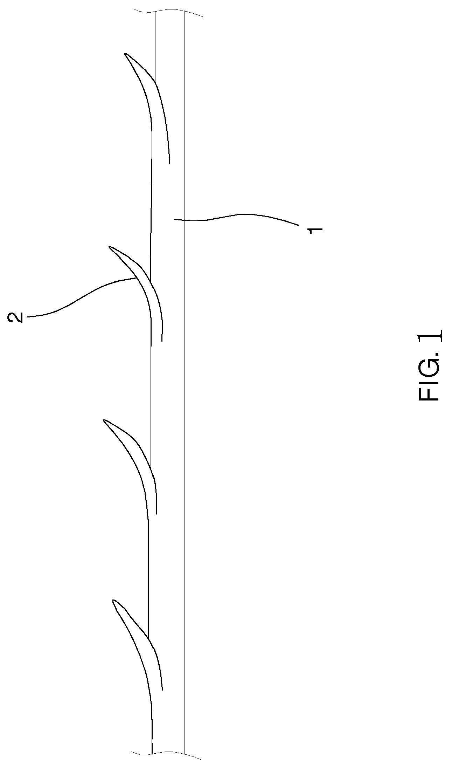

The conventional surgical sutures for thread lift surgery mainly include the following two types. One is commonly known as feather lift (shown in FIG. 1), which is manufactured by cutting or etching to form many barbs 2 on the surface of a thread 1. In this sense, the barbs 2 can lift the dermal tissue when the thread 1 penetrating the subcutaneous tissue of the skin. However, the process of cutting or etching may destroy the structure of the thread 1, which may decrease the strength and toughness of the thread 1, such that the thread 1 is prone to break and disintegrate.

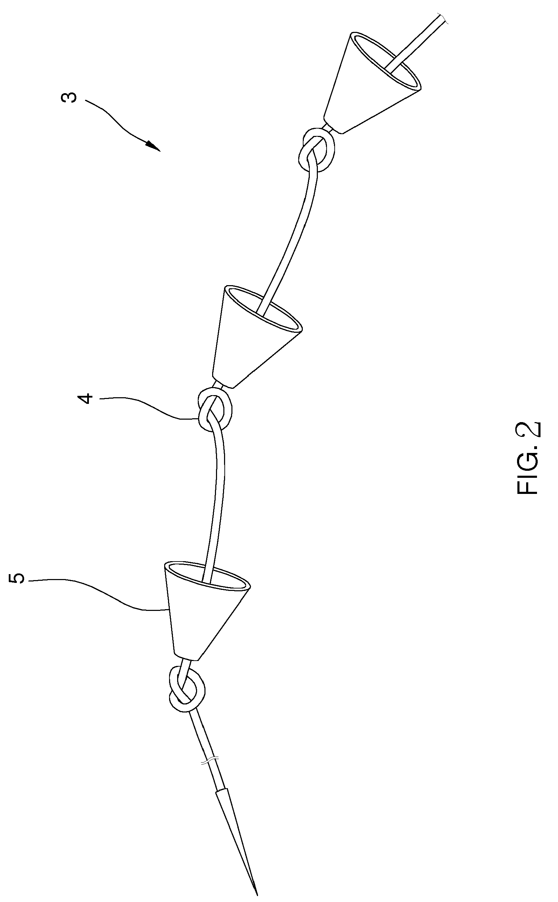

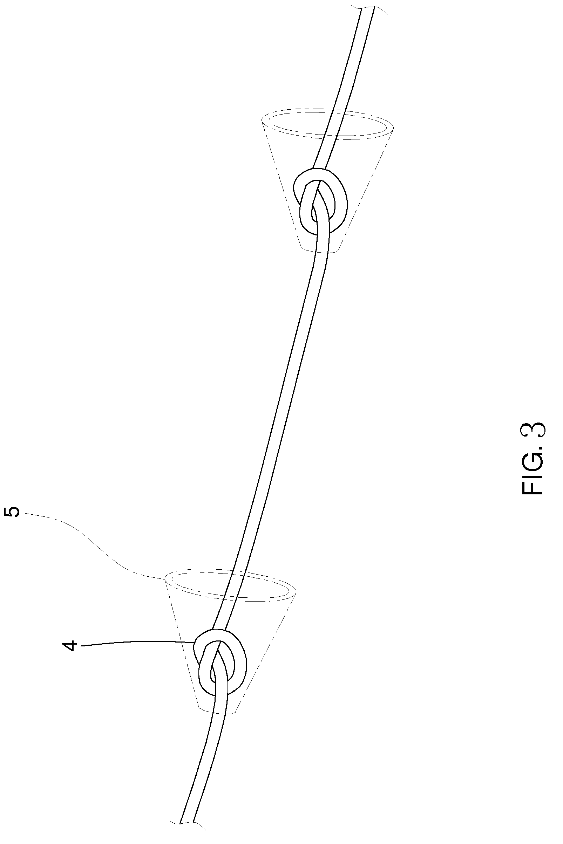

The conventional surgical sutures 3 illustrated in FIG. 2 and FIG. 3 is the other one, forming a plurality of knots 4 on a thread thereof, and including a plurality of cones 5, wherein each of the cones 5 is located between two adjacent knots 4. After the surgical sutures 3 penetrating the skin, each of the cones 5 would move to and engage with the corresponding knot 4. However, there are several problems for the surgical sutures 3, as described below.

The first problem is, because of the high toughness of the thread, it is not easy to tie the knots in the thread.

Next, it's not easy to keep the distance between any two adjacent knots 4 consistent during the manufacturing process.

Another problem is that the unstable knots may be deviated or released due to external forces such as vibration during transportation.

Finally, the highly-tough thread would be deformed after winding, which brings inconvenience to the operation, as well as significantly reduces the lifting effect.

The abovementioned problems are more significant for threads with greater diameters. In more details, according to the United States Pharmacopeia (U.S.P.), the commonly used USP designation of the surgical suture 3 is between 2-0 to 3-0; if the diameter of the thread is greater than that, the extremely strong and tough thread is unable to be tied in knots, and is therefore unable to regulate the position of the cones 5. In other words, the diameter of the conventional surgical suture is limited, resulting in a restriction on the metabolic rate of the thread.

In conclusion, problems of the conventional surgical sutures remain unsolved.

## BRIEF SUMMARY OF THE INVENTION

In view of the above, the primary objective of the present invention is to provide a surgical suture which is not easily deformed, convenient to operate, and the manufacturing process thereof is simple.

The present invention provides a surgical suture including a hollow cone and a thread. The cone has a front opening and a rear opening at two opposite ends thereof, wherein a diameter of the front opening is smaller than a diameter of the rear opening. The thread passes through the front opening and the rear opening of the cone. The thread integrally includes at least two protrusions thereon, and the protrusions are separated from each other by a certain distance. Each of the protrusions has a width in a radial direction of the thread, wherein the width is greater than a diameter of the thread, and is smaller than the diameter of the rear opening.

Whereby, with the protrusions, the cones can be located on the corresponding positions of the thread without deviation. Additionally, the surgical suture is not easily deformed, and is convenient to operate.

## BRIEF DESCRIPTION OF THE SEVERAL VIEWS OF THE DRAWINGS

The present invention will be best understood by referring to the following detailed description of some illustrative embodiments in conjunction with the accompanying drawings, in which

FIG. 1 is a schematic diagram of one of the conventional surgical sutures;
FIG. 2 is a schematic diagram of another type of the conventional surgical sutures;
FIG. 3 is a schematic diagram of the conventional surgical suture in FIG.2 , showing another state of the surgical suture;
FIG. 4 is a top view of a first preferred embodiment of the present invention, showing the surgical suture;
FIG. 5 is a partial enlarged view of the first preferred embodiment;
FIG. 6 is a schematic diagram of the first preferred embodiment;
FIG. 7 is a lateral view of one of the cones of a second preferred embodiment;
FIG. 8 is a bottom view of one of the cones of the second preferred embodiment;

FIG. 9 is a lateral view of one of the cones of a third preferred embodiment;

FIG. 10 is a bottom view of one of the cones of the third preferred embodiment.

## DETAILED DESCRIPTION OF THE INVENTION

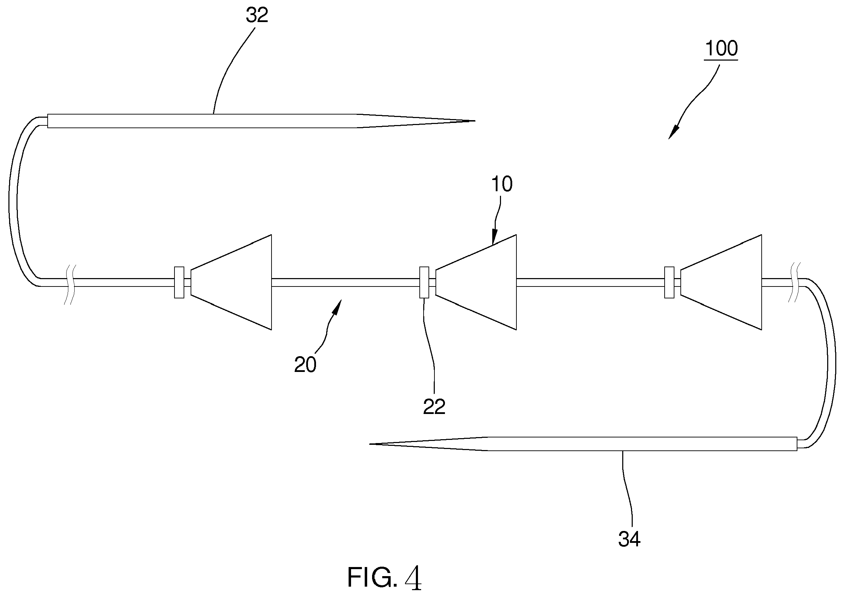

As shown in FIG. 4 to FIG. 6, the surgical suture 100 includes a cone 10 and a thread 20.

The cone 10 is hollow, and has a front opening 12 and a rear opening 14 at two opposite ends thereof. A diameter of the front opening 12 is defined as D1, and a diameter of the rear opening 14 is defined as D2. The surgical suture 100 satisfies D1< D2. The front opening 12 communicates with the rear opening 14, wherein the inner diameter of the cone 10 tapered off from the rear opening 14 to the front opening 12.

The thread 20 passes through the front opening 12 and the rear opening 14 of the cone 10. Both ends of the thread 20 are respectively connected to a needle 32 and 34 in the first preferred embodiment, wherein the two needles 32 and 34 are long and straight. However, in other embodiments, the needles could be change to curved needles or short needles on demand.

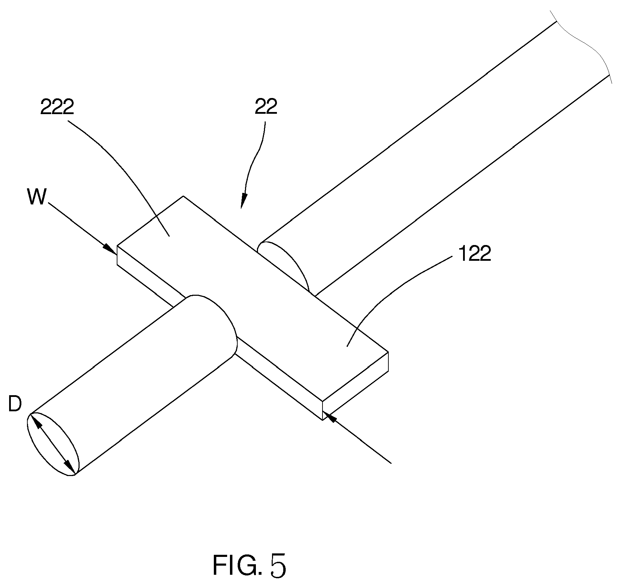

In addition, the thread 20 integrally includes at least two protrusions 22 thereon, and the protrusions 22 are separated from each other by a certain distance. For explanatory need, the thread 20 includes three protrusions 22 in the first preferred embodiment, wherein the protrusions 22 are made by extruding, compressing, or punching the thread 20. As illustrated in FIG. 5, each of the protrusions 22 has two side portions 221 and 222 at two opposite sides of the protrusion 22.

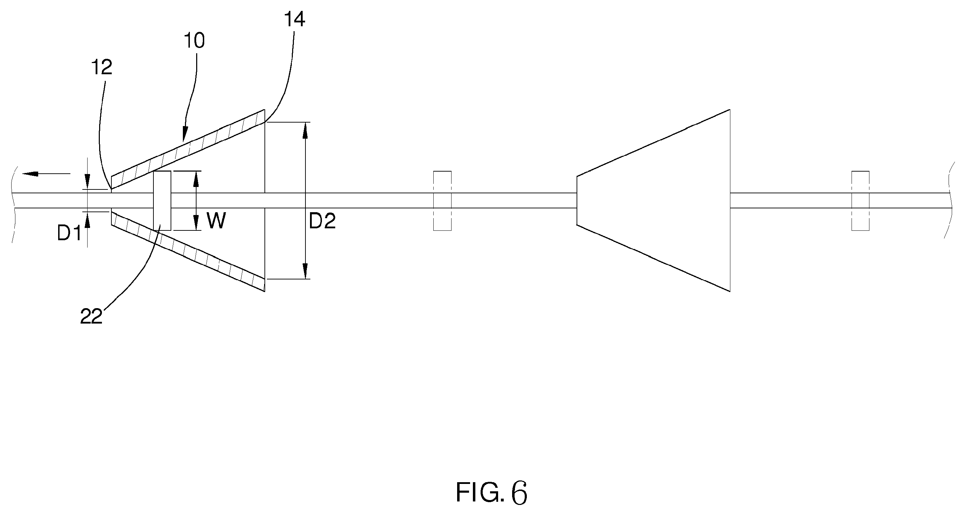

As shown in FIG. 6, when the thread 20 is pulled, or the cone 10 is moving relative to the thread 20, the protrusions 22 could move inside the corresponding cone 10 through the rear opening 14 thereof, and thus abut against the inner wall of the cone 10 for positioning the cones 10 on the thread 20. Additionally, the design of the diameter of the thread 20 and the width of the protrusions 22 satisfies $1.5D \leq W \leq 8D$, where D is the diameter of the thread 20, and W is the width of each of the protrusions 22. The first preferred embodiment satisfies $W = 3D$, such that the strength of the thread 20 is high enough, and also the width of the protrusions 22 is large enough to be connected to the cones 10.

In addition, the first preferred embodiment also satisfies D1< W< D2 for positioning the cones 10 on the thread 20 by the protrusions 22.

Either or both of the cones 10 and the thread 20 are made of a biodegradable material for being degraded or digested after inserted into human bodies, and thus being absorbed or released from the human bodies. In more details, the biodegradable materials includes bio-absorbable bioceramics and degradable polymers, wherein the bio-absorbable bioceramics includes calcium carbonate, tricalcium phosphate, and calcium sulfate; the degradable polymers includes materials from natural sources and synthetic materials, wherein the materials from natural sources includes collagen, starch, cellulose, gelatin, chitin, and hyaluronic acid; the synthetic materials includes polyhydroxyalkanoates (PHA), polycaprolactone (PCL), polyglycolic acid (PGA), poly(lactide-co-glycolide) (PLGA), and polylactic acid (PLA). The biodegradable materials are not limited to be the examples mentioned above, but also may be other compounds in other embodiments.

Especially, the surgical suture 100 includes the protrusions 22 manufactured by high pressure instead of knots. Therefore, the diameter of the thread 20 could be greater to increase the strength of the thread 20 for a long metabolic period, which provides a lasting effect of lifting.

In the preferred embodiments, the diameter of the thread 20 would not be limited, and could between 0.1 mm to 1 mm. In other words, the USP designation of the surgical suture 100 is between 6-0 to 1,2, or more, depending on the demand.

In another preferred embodiment, both the cones and the thread are made of biodegradable materials. However, the materials of the cones and the thread could be non-biodegradable according to the demand.

On the other hand, during the manufacturing process of the first preferred embodiment, the cones are fitted around the thread and are separated from each other at first. Next, through extruding, compressing, or punching the thread, the protrusions are formed between two adjacent cones to separate the cones. With such structure, the surgical suture is adapted to be massively produced with a high yield.

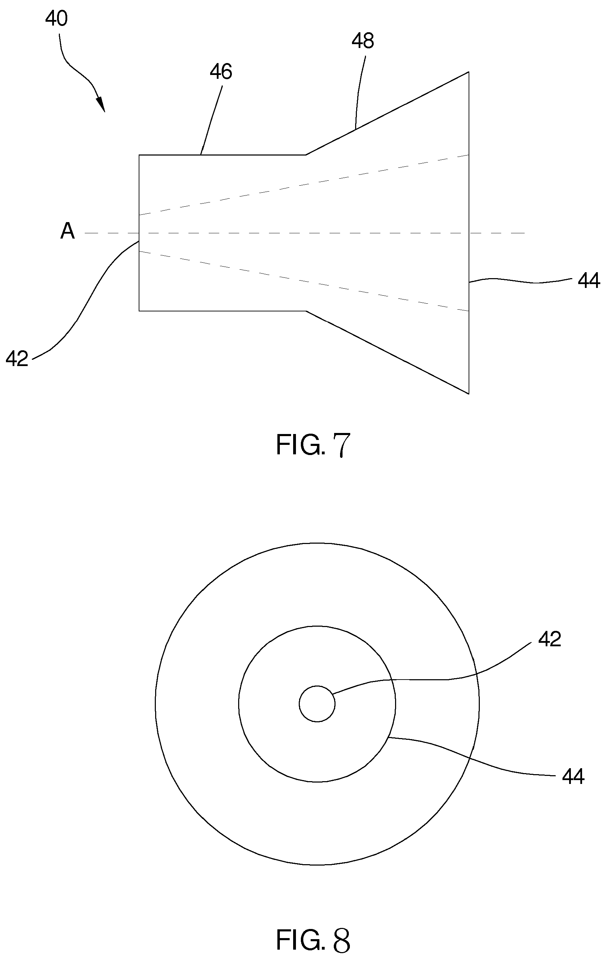

Furthermore, for improving the surgery efficiency, the cone 40 of a second preferred embodiment as shown in FIG. 7 and FIG. 8 also has a front opening 42 and a rear opening 44. The difference between the cone 40 and the cone 10 is that the cone 40 has a first section 46 and a second section 48 neighboring each other, and both of which have an axis A. The first section 46 has the front opening 42, and the second section 48 has the rear opening 44. A slope of the outer surface of the first section 46 relative to the axis A is defined as m1, and a slope of the outer surface of the second section 48 relative to the axis A is defined as m2; the surgical suture in the second preferred embodiment satisfies $m1 \leq m2$. With such design, the cone 40 can not only penetrate human bodies with lower resistance, which increase the efficiency of penetrating, but also prolong the metabolic period with a bigger volume.

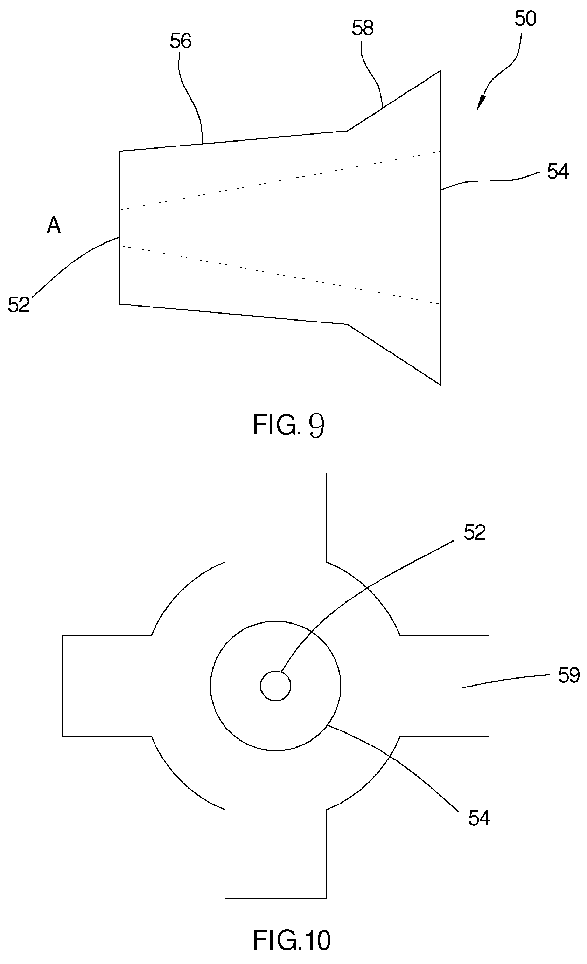

In additional, the cone 50 illustrated in FIG. 9 and FIG. 10 also has a front opening 52 and a rear opening 54, wherein the cone 50 has a first section 56 and a second section 58 neighboring each other, and both of which have an axis A. The first section 56 has the front opening 52, and the second section 58 has the rear opening 54. The slopes of the outer surface of the first section 56 and the outer surface of the second section 58 are different as well. Especially, four wings 59 are provided on an outer surface of the cone 50, wherein the wings 59 are arranged in cruciform symmetry to further increase the lifting effect after the cones 50 penetrating human bodies. However, the number of the wings is not limited to be 4; in other embodiments, two or three wings are provided on an outer surface of the cone to divide the outer surface into many parts.

In addition, the number of the protrusions 22 on the thread could be changed according to the number of the cones 10. On the other hand, the manufacturing method of the protrusions is not limited to high pressure; for example, the thread and the protrusions of the thread can be made by 3D printing.

In another preferred embodiment, the width of the protrusions are greater than the diameter of the rear opening. In more details, the protrusions are elastic and deformable to enter the cones through the corresponding rear openings for engaging with the cones.

It must be pointed out that the embodiments described above are only some preferred embodiments of the present invention. All equivalent structures which employ the concepts disclosed in this specification and the appended claims should fall within the scope of the present invention.

**Claims**

1. A surgical suture (100), comprising:

   a hollow cone (10, 40, 50) having a front opening (12, 42, 52) and a rear opening (14, 44, 54) at two opposite ends thereof, wherein a diameter (D1) of the front opening (12, 42, 52) is smaller than a diameter (D2) of the rear opening (14, 44, 54);
   a thread (20) passing through the front opening (12, 42, 52) and the rear opening (14, 44, 54) of the cone (10, 40, 50), wherein the thread (20) integrally comprises at least two protrusions (22) thereon, and the protrusions (22) are separated from each other by a certain distance; each of the protrusions (22) has a width (W) in a radial direction of the thread (20), wherein the width (W) is greater than a diameter (D) of the thread (20), and is smaller than the diameter (D2) of the rear opening (14, 44, 54).

2. The surgical suture (100) of claim 1, wherein the protrusions (22) are made by extruding, compressing, or punching the thread (20).

3. The surgical suture (100) of claim 2, wherein each of the protrusions (22) has two side portions (221, 222) at two opposite sides of the protrusion (22).

4. The surgical suture (100) of claim 1, satisfying:

$$1.5D \leq W \leq 8D;$$

where D is the diameter (D) of the thread (20), and W is the width (W) of each of the protrusions (22).

5. The surgical suture (100) of claim 4, satisfying:

$$W = 3D.$$

6. The surgical suture (100) of claim 1, wherein the diameter (D1) of the front opening (12, 42, 52) is between 0.2 mm to 0.4 mm; the diameter (D2) of the rear opening (14, 44, 54) is between 0.5 mm to 2 mm.

7. The surgical suture (100) of claim 1, wherein either or both of the thread (20) and the cone (10, 40, 50) are made of a biodegradable material.

8. The surgical suture (100) of claim 1, satisfying:

$$0.2 \text{ mm} \leq D \leq 1 \text{ mm};$$

where D is the diameter (D) of the thread (20).

9. The surgical suture of claim 1, wherein the cone (40, 50) has a first section (46, 56) and a second section (48, 58), and both of which have an axis (A); wherein the surgical suture satisfies:

$$m1 \leq m2;$$

where m1 is a slope of the outer surface of the first section (46, 56) relative to the axis (A), and m2 is a slope of the outer surface of the second section (48, 58) relative to the axis (A).

10. The surgical suture of claim 1, further comprising a plurality of wings (59) provided on an outer surface of the cone (50).

FIG. 1

FIG. 2

EP 3 225 175 A1

FIG. 3

FIG. 4

FIG. 5

FIG. 6

FIG. 7

FIG. 8

FIG. 9

FIG.10

Europäisches
Patentamt

European
Patent Office

Office européen
des brevets

# EUROPEAN SEARCH REPORT

Application Number

EP 16 16 3498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2007/038249 A1 (KOLSTER ALWIN [US]) 15 February 2007 (2007-02-15) | 1-5,8,9 | INV. A61B17/06 |
| Y | * figures 3, 20 * * paragraph [0068] * | 6,7,10 | ADD. A61B17/00 |
| Y | GB 2 527 689 A (JUBURI FALAH AL [IQ]) 30 December 2015 (2015-12-30) | 6,7,10 | |
| A | * figures 2-4, 6 * * page 1, paragraph 5 - paragraph 6 * * page 4, paragraph 9 - last paragraph * | 1 | |
| X | US 5 370 661 A (BRANCH THOMAS P [US]) 6 December 1994 (1994-12-06) * figures 3-6, 10 * * column 5, line 27 - line 68 * * column 8, line 9 - line 13 * | 1,3-6, 8-10 | |
| X | WO 2004/112585 A2 (MEDTRONIC VASCULAR INC [US]; HUYNH RANY [US]; BLOOM ELIOT [US]; LEMMON) 29 December 2004 (2004-12-29) * figures 12, 13 * * paragraph [0043] * | 1-6,8,9 | TECHNICAL FIELDS SEARCHED (IPC) |
| A | US 2012/101526 A1 (BENNETT WILLIAM F [US]) 26 April 2012 (2012-04-26) * figure 1 * * paragraph [0396] - paragraph [0397] * | 1,9 | A61B |

The present search report has been drawn up for all claims

| Place of search | Date of completion of the search | Examiner |
|---|---|---|
| Munich | 21 June 2016 | Etienne, Nicolas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                          

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 16 16 3498

This annex lists the patent family members relating to the patent documents cited in the above-mentioned European search report.
The members are as contained in the European Patent Office EDP file on
The European Patent Office is in no way liable for these particulars which are merely given for the purpose of information.

21-06-2016

| Patent document cited in search report | | | Publication date | Patent family member(s) | | | Publication date |
|---|---|---|---|---|---|---|---|
| US 2007038249 | A1 | | 15-02-2007 | AU | 2007284933 | A1 | 21-02-2008 |
| | | | | BR | PI0716640 | A2 | 02-04-2013 |
| | | | | CA | 2695924 | A1 | 21-02-2008 |
| | | | | CN | 101500495 | A | 05-08-2009 |
| | | | | CN | 103251433 | A | 21-08-2013 |
| | | | | EP | 2051747 | A2 | 29-04-2009 |
| | | | | HK | 1131873 | A1 | 13-09-2013 |
| | | | | JP | 5506383 | B2 | 28-05-2014 |
| | | | | JP | 2010500102 | A | 07-01-2010 |
| | | | | JP | 2014195649 | A | 16-10-2014 |
| | | | | JP | 2015131124 | A | 23-07-2015 |
| | | | | KR | 20090035692 | A | 10-04-2009 |
| | | | | MY | 148454 | A | 30-04-2013 |
| | | | | NZ | 574434 | A | 24-02-2012 |
| | | | | RU | 2009108331 | A | 20-09-2010 |
| | | | | US | 2007038249 | A1 | 15-02-2007 |
| | | | | US | 2009182375 | A1 | 16-07-2009 |
| | | | | WO | 2008020937 | A2 | 21-02-2008 |
| GB 2527689 | A | | 30-12-2015 | NONE | | | |
| US 5370661 | A | | 06-12-1994 | US | 5370661 | A | 06-12-1994 |
| | | | | US | 5372146 | A | 13-12-1994 |
| | | | | US | 5520691 | A | 28-05-1996 |
| WO 2004112585 | A2 | | 29-12-2004 | EP | 1648346 | A2 | 26-04-2006 |
| | | | | JP | 2007535335 | A | 06-12-2007 |
| | | | | US | 2006282161 | A1 | 14-12-2006 |
| | | | | WO | 2004112585 | A2 | 29-12-2004 |
| US 2012101526 | A1 | | 26-04-2012 | EP | 2632345 | A1 | 04-09-2013 |
| | | | | US | 2012101524 | A1 | 26-04-2012 |
| | | | | US | 2012101526 | A1 | 26-04-2012 |
| | | | | US | 2015032156 | A1 | 29-01-2015 |
| | | | | WO | 2012058301 | A1 | 03-05-2012 |

EPO FORM P0459

For more details about this annex : see Official Journal of the European Patent Office, No. 12/82